# FASCICULE DE BREVET EUROPEEN

(11) **EP 2 079 460 B1**
(45) Date de publication et mention de la délivrance du brevet: **21.11.2018**
(21) Numéro de dépôt: 07858625.2
(22) Date de dépôt: 19.10.2007
(51) Int. Cl.: A61K 31/05, A61K 8/60, A61Q 19/06, A61Q 19/08

(54) **UTILISATION COSMETIQUE PAR VOIE ORALE DE LA GLUCOSAMINE EVENTUELLEMENT ASSOCIEE A AU MOINS UN COMPOSE POLYPHENOL**
KOSMETISCHE ORALE VERWENDUNG VON GLUCOSAMIN WAHLWEISE IN KOMBINATION MIT MINDESTENS EINER POLYPHENOLVERBINDUNG
COSMETIC ORAL USE OF GLUCOSAMINE OPTIONALLY IN COMBINATION WITH AT LEAST ONE POLYPHENOL COMPOUND

(30) Priorité: 20.10.2006 FR 0654425; 20.10.2006 FR 0654426
(43) Date de publication de la demande: 22.07.2009
(73) Titulaire: NUTRICOS Technologies, 92117 Clichy Cedex (FR)
(72) Inventeur: PICCIRILLI, Antoine, 86000 Poitiers (FR); MANISSIER, Patricia, 92300 Levallois-perret (FR); MONTASTIER, Christiane, 75016 Paris (FR)
(74) Mandataire: Nony
(86) Numéro de dépôt international: PCT/FR2007/052202
(87) Numéro de publication internationale: WO 2008/047061

(56) Documents cités:
- EP-A- 1 075 836
- WO-A-01/13865
- WO-A-02/080708
- WO-A-2004/041199
- WO-A-2005/053710
- JP-A- 2004 083 432
- JP-A- 2004 083 434
- US-A- 5 804 594
- US-A1- 2006 105 989
- US-B1- 6 333 304
- "INNEOV CELLULITE,UNE FORMULE EXCLUSIVE" INTERNET ARCHIVE WAYBACK MACHINE, [Online] 4 septembre 2006 (2006-09-04), XP002478664 Extrait de l'Internet: URL:http://www.inneov.com/> [extrait le 2008-04-29]
- "Murad Cosmetics" INTERNET CITATION, [Online] 1997, XP002436922 Extrait de l'Internet: URL:http://www.joiedevie.com/products-mura d3.htm> [extrait le 2007-06-20]
- NANCI MC ARDLE: "The inside track to beauty" INTERNET CITATION, [Online] 1996, XP002436923 Extrait de l'Internet: URL:http://www.happi.com/articles/2005/07/ the-inside-track-to-beauty.php> [extrait le 2007-06-08]
- BLAZSO G ET AL: "Pycnogenol Accelerates Wound Healing and Reduces Scar Formation" PHYTOTHERAPY RESEARCH, JOHN WILEY & SONS LTD. CHICHESTER, GB, vol. 18, 2004, pages 579-581, XP002418682 ISSN: 0951-418X
- MARY.C.OTTOLINI ET AL.: "Complementary and alternative medicine use among children in the washington , DC area" AMBULATORY PEDIATRICS, vol. 1, no. 2, mars 2001 (2001-03), - avril 2001 (2001-04) pages 122-125, XP005385833
- MURAD H ET AL: "THE EFFECT OF AN ORAL SUPPLEMENT CONTAINING GLUCOSAMINE, AMINO ACIDS, MINERALS, AND ANTIOXIDANTS ON CUTANEOUS AGING: A PRELIMINARY STUDY" JOURNAL OF DERMATOLOGICAL TREATMENT, BASINGSTOKE, GB, vol. 12, no. 1, mars 2001 (2001-03), pages 47-51, XP008041535 ISSN: 0954-6634 cité dans la demande
- MANTLE D ET AL: "A novel therapeutic strategy for Ehlers-Danlos syndrome based on nutritional supplements" MEDICAL HYPOTHESES, EDEN PRESS, PENRITH, US, vol. 64, no. 2, 2005, pages 279-283, XP004684526 ISSN: 0306-9877
- DATABASE GNPD [Online] MINTEL; July 2005 (2005-07), "Slimming Help Capsules with Green Tea and Grape", Database accession no. 379017

## Description

La présente invention se situe dans le domaine des compléments alimentaires ou des aliments fonctionnels destinés au soin de la peau.

La peau humaine est constituée de trois compartiments à savoir un compartiment superficiel, l'épiderme, le derme et un compartiment profond, l'hypoderme. Ce dernier compartiment est essentiellement constitué d'un type de cellules spécialisées dans l'accumulation et le stockage des graisses, les adipocytes. L'hypoderme est le réservoir énergétique de l'organisme.

L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué par la peau.

Le derme fournit à l'épiderme un support solide. C'est également son élément nourricier. Il est principalement constitué de fibroblastes et d'une matrice extracellulaire composée elle-même principalement de collagène, d'élastine et d'une substance, dite substance fondamentale, composants synthétisés par le fibroblaste.

On y trouve aussi des leucocytes, des mastocytes ou encore des macrophages tissulaires. Il est également traversé par des vaisseaux sanguins et des fibres nerveuses. Dans une peau normale, c'est à dire non pathologique ni cicatricielle, le fibroblaste est à l'état quiescent, c'est à dire non prolifératif.

Ce sont les fibres de collagène qui assurent la solidité du derme. Les fibres de collagène sont constituées de fibrilles scellées les unes aux autres, formant ainsi plus de dix types de structures différentes. La solidité du derme est en grande partie due à l'enchevêtrement des fibres de collagène tassées les unes contre les autres en tous sens. Les fibres de collagène participent à l'élasticité et à la tonicité de la peau et/ou des muqueuses.

Les fibres de collagène sont constamment renouvelées mais ce renouvellement diminue avec l'âge ce qui entraîne un amincissement du derme. Cet amincissement du derme est également dû à des causes pathologiques comme par exemple l'hypersécrétion d'hormones corticoïdes, certaines pathologies ou encore des carences vitaminiques (cas de la vitamine C dans le scorbut). Il est également admis que des facteurs extrinsèques comme les rayons ultraviolets, le tabac ou certains traitements (Glucocorticoïdes, vitamine D et dérivés par exemple) ont également un effet sur la peau et sur son taux de collagène.

Divers facteurs entraînent la dégradation du collagène, avec toutes les conséquences que l'on peut envisager sur la structure et/ou la fermeté de la peau et/ou des muqueuses.

Bien que très résistantes, les fibres de collagène sont sensibles à certaines enzymes appelées collagénases. Une dégradation des fibres de collagène entraîne l'apparence de peau molle et ridée que l'être humain, préférant l'apparence d'une peau lisse et tendue, cherche depuis toujours à combattre.

Les collagénases font partie d'une famille d'enzymes appelées métalloprotéinases (MMPs) qui sont elles-mêmes les membres d'une famille d'enzymes protéolytiques (endoprotéases ou endopeptidases) qui possèdent un atome de zinc coordonné à 3 résidus cystéine et une méthionine dans leur site actif et qui dégradent les composants macromoléculaires de la matrice extracellulaire et des membranes basales à pH neutre (collagène, élastine, etc). Très largement répandues dans le monde vivant, ces enzymes sont présentes, mais faiblement exprimées, dans des situations physiologiques normales comme la croissance des organes et le renouvellement des tissus.

Leur surexpression chez l'homme et leur activation est liée à de nombreux processus, parfois pathologiques, qui impliquent la destruction et le remodelage de la matrice. Cela entraîne soit une résorption non contrôlée de la matrice extracellulaire, soit inversement l'installation d'un état de fibrose.

La famille des métalloprotéinases est constituée de plusieurs groupes bien définis basés sur leurs ressemblances en terme de structure et de spécificité de substrat. Parmi ces groupes, on peut citer les collagénases destinées à dégrader les collagènes fibrillaires (MMP-1 ou collagénase interstitielle, MMP-8 ou collagénase de neutrophile, MMP-13 ou collagénase 3), les gélatinases qui dégradent le collagène de type IV ou toute forme de collagène dénaturé (MMP-2 ou gélatinase A (72 kDa), MMP-9 ou gélatinase B (92 kDa)).

Les stromélysines (MMP-3) présentent quant à elles un large spectre d'activité s'adressant aux protéines de la matrice extracellulaire telles que les glycoprotéines (fibronectine, laminine), les protéoglycannes, etc., ou encore les métalloprotéinases membranaires. Ces dernières n'ont pas le rôle anti-collagénase des métalloprotéinases rapportées ci-dessus.

En outre, certains protéoglycannes tels que que ceux qui appartiennent à la famille des Small Leucine-Rich Proteoglycans (SLRP), constituent une cible intéressante en vue de prévenir les effets négatifs du vieillissement et la déterioration des propriétés mécaniques de la peau. Ces SLRP sont en effet directement impliqués dans la fibrillogénèse et l'hydratation des espaces périfibrillaires. Les SLRP contribuent notamment à accroître la biodisponibilité de certains facteurs de croissance tels que le TGF-β : parmi les SLRP, on peut citer la décorine, le lumican, la fibromoduline, le biglycan. Par ailleurs, certaines observations immunohistochimiques démontrent une diminution de l'accumulation de biglycan dans la peau âgée. De même, la diminution marquée de lumican et de fibromoduline induit une alteration de la fibrillogénèse du collagène ainsi qu'une perturbation de l'architecture fibrillaire. Par conséquent, les protéoglycannes de la famille des SLRP jouent un rôle fondamental dans l'organisation architecturale des structures de la peau et donc dans la régulation de la fermeté cutanée.

Enfin, les SLRPs sont non seulement sensibles à l'action des MMPs, mais également à l'action protéolitique des agrécanases ou ADAMTS (A Disentegrin And Metalloprotease with Thrombospondin type I repeat). Certains membres de cette nouvelle famille de metalloprotéases, en particulier ADAMTS 1 et 4, ont été identifiés au niveau de la peau, et ADAMTS4 est connu pour cliver la décorine.

L'exposition prolongée aux rayonnements ultraviolets, particulièrement aux rayonnements ultraviolets de type A et/ou B, a pour effet une stimulation de l'expression des collagénases, particulièrement de la MMP-1. C'est là une des composantes du vieillissement cutané photo-induit.

Par ailleurs à la ménopause, les principales modifications concernant le derme sont une diminution du taux de collagène et de l'épaisseur dermique. Cela entraîne chez la femme ménopausée un amincissement de la peau et/ou des muqueuses. La femme ressent alors une sensation de « peau papyracée » ou de peau qui tire et l'on constate une accentuation des fines rides et ridules de surface. La peau présente un aspect rugueux à la palpation. Enfin la peau présente une souplesse diminuée.

Enfin, chez le sujet en surpoids et plus particulièrement lors de la prise de poids, les adipocytes ont tendance à augmenter rapidement de volume (stockage de quantités croissantes de lipides). Les lobules graisseux se distendent alors peu à peu pour conduire à la formation de travées conjonctives, parallèles entre elles et perpendiculaires à la surface cutanée. La forte pression exercée par les adipocytes sur le derme provoque rapidement une déformation de la surface de la peau. Au plan cutané, ce phénomène dit de cellulite, se traduit par un aspect capitonné donnant par endroit les signes de « peau d'orange ». Enfin, au plan clinique, la cellulite se traduit par une modification de la texture des tissus sous-cutanés et superficiels, caractérisée en particulier par :
- une peau dans sa globalité plus épaisse,
- une peau plus consistante,
- une peau plus sensible pouvant même en fonction du stade d'avancement de la cellulite être douloureuse à la palpation, et/ou
- des tissus cutanés moins mobiles du fait de la perte d'adhérence et de cohésion des couches profondes de la peau.

Aussi, ce phénomène est plus visible chez les femmes car elles ont une peau plus fine avec des travées conjonctives présentant une structure verticale, qui par contraste chez l'homme, ont une structure oblique et inter croisée.

La cellulite, qui est souvent aggravée par le surpoids et l'obésité, est surtout localisée au niveau du bassin et des membres inférieurs (cellulite en « culotte de cheval » ou « pantalon de zouave »). Ces modifications peuvent aussi conduire à des déformations cicatricielles définitives.

L'hypertrophie du tissu adipeux s'accompagne au niveau dermique d'une mise sous tension des réseaux de fibres, entraînant une altération fonctionnelle des cellules résidentes. En effet, cette hypertension entrave les échanges cellulaires, la circulation veineuse et lymphatique par compression de capillaires, si bien que le phénomène s'auto-entretient. A terme, les fibres dégénèrent et la peau perd ses structures fondamentales.

Au plan biologique, lorsque les fibroblastes sont soumis à une tension tissulaire normale, ils synthétisent activement du collagène, de l'élastine, des glycosminoglycanes, molécules fondamentales qui contribuent à renforcer les tissus de soutien de la peau. De façon analogue, les adipocytes surchargés de lipides exercent aussi une tension sur le derme, entraînant une surproduction de collagène jusqu'à la fibrose. Cela se traduit au plan clinique, par une peau plus consistante et tendue.

En revanche, lors d'une perte de poids et notamment au cours des régimes amincissants, le déstockage rapide des adipocytes entraîne une diminution importante de la tension exercée par l'hypoderme sur les tissus de soutien. Par conséquent, le derme n'étant plus sous tension, le tissu conjonctif perd progressivement de sa cohésion : perte d'attache des fibroblastes au collagène, diminution de la quantité de néocollagène, distension des fibres d'élastine, dépolymérisation des protéoglycannes, .... Dès lors, les fibroblastes en moins grande interaction avec les fibres de la matrice extracellulaire, ne reçoivent plus de leur environnement, les signaux d'activité et de réparation qui commandent la synthèse des macromolécules fondamentales du derme. De plus, les fibroblastes ne recevant plus de signaux de leur environnement fibrillaire sécrètent des métalloprotéases matricielles (MMPs), enzymes entraînant la dégradation des structures fibreuses. Ce ralentissement marqué du métabolisme des fibroblastes, ainsi que la dégradation des fibres par les MMPs, se traduisent par voie de conséquence, par une altération des propriétés viscoélastiques ou biomécaniques de la peau (perte de fermeté, de tonicité, d'élasticité, ...).

On comprend alors à la lecture de ce qui précède l'importance du collagène et des glycosaminoglycanes dans la structure des tissus, particulièrement de la peau et/ou des muqueuses, et l'importance qu'il y a à combattre sa dégradation pour ainsi lutter contre le vieillissement, qu'il soit chronobiologique ou photo-induit, et ses conséquences, notamment sur l'amincissement du derme et/ou la dégradation des fibres de collagène, cette dernière conséquence entraînant une perte de fermeté cutanée et en particulier l'apparence de peau molle contre laquelle l'objet de la présente invention est précisément de lutter.

La présente invention s'intéresse plus particulièrement à la prévention et/ou au traitement par voie orale de la perte de fermeté cutanée, induite par la ménopause.

On note que dans la littérature, une des voies décrites pour lutter contre la cellulite consiste à stimuler le lipolyse, par exemple en inhibant la phosphodiestérase ou en activant des β-adrénorécepteurs.

L'administration orale de la glucosamine est principalement connue dans le traitement de l'arthrose, notamment sous forme de complément alimentaire.

Le document « The effect of an oral supplement containing glucosamine, amino acids, minerals, and antioxydants on cutaneous aging : a preliminary study », H. MURAD and Michael TABIBIAN, Journal of Dermatological Treatment (2001)12, 47-51 rapporte une étude sur un groupe de volontaires traités par voie orale par un produit contenant de multiples ingrédients, à savoir de la D-glucosamine acétylée, du sulfate de glucosamine, de la L-proline, de la L-lysine, du manganèse, du cuivre, du zinc de la quercetine et un extrait de pépins de raisins. Les résultats semblent montrer un effet sur le nombre diminué de rides, observées après traitement.

L'utilisation par voie topique de l'acétylglucosamine est par ailleurs connue à titre de conditionneur de la peau. Aussi, cette même utilisation a été décrite pour combattre la cellulite, notamment dans la publication WO 01/13865 A1. L'acétylglucosamine est également un ingrédient dans certaines crèmes, notamment hydratantes, destinées à améliorer l'apparence cutanée.

Le document EP 1 075 836 décrit une composition de soin de la peau comprenant de la N-acétylglucosamine à titre d'agent actif.

Le document JP 2004-083432 décrit un inhibiteur d'élastase destiné à améliorer les rides et le vieillissement cutané, comprenant de la glucosamine, ses dérivés ou ses sels.

Enfin, le document JP 2004-083434 décrit un agent pour favoriser la synthèse de collagène comprenant de la glucosamine, ses dérivés ou ses sels.

L'invention a pour objet l'utilisation cosmétique par voie orale de la glucosamine comprenant toutes ses formes salifiées, acétylées et/ou polymériques dans la prévention et/ou le traitement de la perte de la fermeté cutanée induite par la ménopause.

On connaît des traitements topiques permettant de lutter contre les signes cutanés du vieillissement. Toutefois, les actifs topiques préconisés n'agissent pas toujours du fait de leur faible pénétration cutanée, au niveau dermique. En outre, les produits topiques agissent par définition localement sur les zones à traiter, zones sur lesquelles ils
peuvent être inégalement répartis, et nécessitent des applications soignées et répétées. Ils peuvent être dans certains cas à l'origine de réactions secondaires cutanées, voire d'inconfort.

Par opposition, la voie orale présente l'avantage d'agir de façon globale sur l'ensemble de la peau et ce dans ces couches profondes (derme, hypoderme). En effet, la glucosamine et/ou ses métabolites sont alors distribués au sein de la matrice dermique par le biais de la circulation sanguine.

Ainsi, la voie orale ou l'administration par patch présentent également l'avantage d'un mode d'administration rapide et peu contraignant.

Par opposition, la voie topique, du fait de la plus faible pénétration cutanée, ne permet pas toujours de déployer toutes les propriétés des actifs, au niveau dermique.

En outre, les produits topiques agissent par définition localement sur les zones à traiter, zones sur lesquelles ils peuvent être inégalement répartis, et nécessitent des applications soignées et répétées. Ils peuvent être dans certains cas à l'origine de réactions secondaires cutanées, voire d'inconfort.

Les inventeurs ont également plus particulièrement découvert que l'administration par voie orale de l'association de glucosamine et d'au moins un composé polyphénol en particulier un composé polyphénol issu d'écorce de pin présente une activité bénéfique sur la peau. Les inventeurs ont aussi plus particulièrement observé que cette association est avantageuse lorsqu'elle est administrée par voie orale notamment sur le maintien et/ou la restauration des propriétés biomécaniques de la peau. Elle permet encore plus particulièrement de maintenir et/ou de restaurer les propriétés d'extensibilité, de tonicité, de fermeté, de souplesse et/ou d'élasticité de la peau et/ou de prévenir et/ou traiter les désordres cutanés induits par la cellulite.

Les composés polyphénols sont notamment connus pour leur fort pouvoir antioxydant et sont couramment utilisés dans des produits cosmétiques. On a également décrit leur rôle dans la prévention des maladies cardiovasculaires par voie orale.

L'utilisation en cosmétique d'extrait maritime d'écorce de pin, notamment commercialisée sous la dénomination Pycnogénol®, est décrite dans le document F. Schönlau, « The cosmeceutical pycnogenol® », Journal of applied cosmetology, vol. 20, n° 4, 2002, pages 241-246.

Ainsi, dans le cadre de la présente invention, la glucosamine peut être associée à au moins un composé polyphénol.

Ce polyphénol peut être un composé polyphénol issu d'écorce de pin.

L'invention décrit également l'utilisation cosmétique par voie orale de l'association de glucosamine et d'au moins un composé polyphénol à titre de mélange d'actifs destiné à maintenir et/ou à restaurer les propriétés biomécaniques de la peau.

Par « propriétés viscoélastiques ou biomécaniques de la peau » dans le cadre de la présente invention, on entend les propriétés d'extensibilité, de tonicité, de fermeté, de souplesse, de densité et/ou d'élasticité de la peau.

Par « signes cutanés du vieillissement », on entend toutes modifications de l'aspect extérieur de la peau dues au vieillissement qu'il soit chronobiologique et/ou extrinsèque, en particulier photo-induit et/ou hormonal, comme par exemple les rides et ridules, la peau flétrie, la peau molle, la peau amincie, la peau terne et sans éclat, le manque d'élasticité et/ou de tonus de la peau, mais également toutes modifications internes de la peau qui ne se traduisent pas systématiquement par un aspect extérieur modifié, comme par exemple toutes dégradations internes de la peau, particulièrement des fibres de collagène, consécutives à une exposition aux rayonnements ultraviolets.

Ce terme est considéré comme équivalent au terme « désordres cutanés induits par le chronovieillissement et/ou le vieillissement extrinsèque et/ou le vieillissement hormonal ».

Il est entendu dans le cadre de la présente invention que « l'utilisation cosmétique par voie orale» recouvre l'utilisation de produits administrés par voie orale, ces produits par exemple sous forme de complément alimentaire ou d'élément fonctionnel comme exposé ci-après pour le cas de la voie orale, produisant un effet, au niveau de la peau, sur le plan esthétique et du confort, ou encore à
visée beauté, par exemple en vue de la protéger, de la maintenir en bon état, d'en modifier l'aspect, et notamment de l'embellir.

### GLUCOSAMINE

La glucosamine est un sucre aminé, notamment d'origine marine, composé d'une base glucose et d'une fonction amine. C'est un sucre simple, de faible masse moléculaire et qui se présente sous la forme de cristaux blancs, solubles dans l'eau, de formule (1) ou (2).

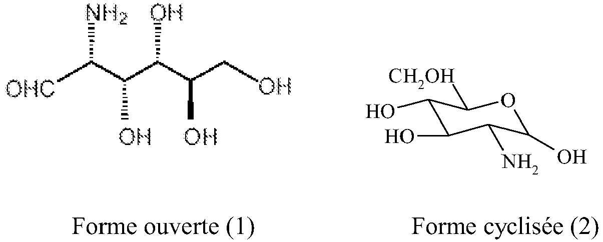

La glucosamine peut être obtenue à partir de la chitine qui est un sucre naturel complexe, biodégradable et tout aussi présent dans la nature que la cellulose.

La chitine est le premier constituant de la carapace (exosquelette) des crustacés tels que le crabe, la crevette ou le homard. C'est donc un polymère marin composé d'unités de glucosamine. Dans la chitine, plus de 60 % de la glucosamine totale est présente sous forme acétylée.

La formule chimique de la chitine est la suivante :

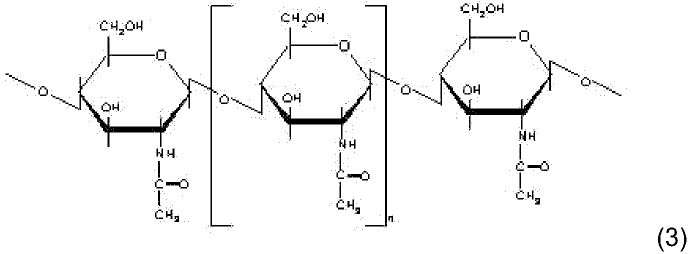

Il existe une autre source de chitine pouvant servir à l'obtention au chlorhydrate de glucosamine. Il s'agit de chitine obtenue de la biomasse produite par fermentation du champignon *A. niger* de la classe des *deuteromycetes*, ordre des *Monoliales*, famille des *Moniliaceae,* genre *Aspergillus* et espèce *niger.*

Dans le commerce, la glucosamine peut être proposée sous différentes formes salines : sulfate, sulfate chloropotassique (2 KCl) sulfate chlorosodique (2NaCl), chlorhydrate (HCl), acétylée ou encore polymérisée (N-acétyl-glucosamine). La nature du sel dépend souvent du procédé d'obtention mis en oeuvre. Toutefois, les formes les plus utilisées et étudiées, le sulfate et le chlorhydrate de glucosamine, correspondent aux sels les plus solubles.

Ainsi, dans le cadre de la présente invention, il est entendu que le terme « glucosamine » comprend toutes ses formes salifiées, acétylées et/ou polymériques. Parmi les formes salifiées de la glucosamine, on peut citer le sulfate, le sulfate chloropotassique, le sulfate chlorosodique et le chlorhydrate.

On rapporte ci-après, à titre illustratif, un exemple de procédé d'obtention.

La glucosamine sous forme sulfatée peut par exemple être préparée à partir des carapaces de crevettes (riches en chitine).

Dans ce procédé d'obtention, la première étape consiste en une hydrolyse acide en présence d'acide chlorhydrique, réalisée sous vide, à 95 °C. Les conditions d'hydrolyse sont dépendantes de la matière première.

Toujours dans ce procédé d'obtention, le milieu réactionnel est ensuite décoloré par le charbon actif qui retient au passage les impuretés organiques absorbables.

Après concentration progressive sous vide, le chlorhydrate de glucosamine obtenu après hydrolyse, cristallise lentement à froid pendant plusieurs heures. Après filtration ce dernier est lavé à l'alcool.

Enfin, la dernière étape de ce procédé d'obtention consiste à neutraliser le chlorhydrate de glucosamine par le sulfate de potassium en phase aqueuse. Le mélange est ensuite évaporé et le solide obtenu séché sous vide à 60 °C pendant plusieurs heures.

Lorsque la chitine issue du champignon précité est utilisée en lieu et place de la chitine issue des carapaces de crevettes, celle-ci subit également une hydrolyse pour produire la glucosamine.

La composition selon l'invention apporte de préférence le composé glucosamine dans une dose journalière allant de 50 mg à 3 g/jour, de préférence de 200 à 2000 mg/jour, et de façon encore plus préférée de 250 à 1500 mg/jour.

De préférence, la glucosamine est présente dans la composition de l'invention dans une teneur allant de 0,0001 à 80 % en poids, de préférence de 1 à 50 % et de façon encore plus préférée de 10 à 20 % en poids par rapport au poids total de la composition.

Comme l'illustre l'exemple 1 qui suit, les inventeurs ont démontré que la glucosamine stimule la synthèse du collagène ainsi que l'expression du récepteur de l'acide hyaluronique CD44.

Notamment, à ce titre, l'utilisation cosmétique par voie orale et/ou parentérale de la glucosamine en tant qu'activateur du métabolisme des fibroblastes ou activateur de la synthèse du collagène et en tant qu'agent promoteur du rétablissement de l'homéostasie épidermique fait également partie de l'invention.

L'exemple 2 illustre de plus une activité anti-dégradative des constituants matriciels du derme du sulfate de glucosamine en ce qu'il réduit le niveau d'expression de la MMP3.

Notamment, de ce fait, la présente invention vise en outre l'utilisation cosmétique de la glucosamine par voie orale et/ou parentérale à titre d'agent d'inhibition de l'expression de la MMP3.

En outre, l'exemple 3 met en évidence un effet stimulateur de la glucosamine sur le cytosquelette des fibroblastes en ce qu'elle permet d'améliorer les propriétés contractiles des fibroblastes, facteur jouant sur la fermeté cutanée.

La présente invention vise aussi l'utilisation cosmétique par voie orale et/ou parentérale à titre d'agent favorisant la contractilité des fibroblastes.

Enfin, l'exemple 4 montre l'effet du sulfate de glucosamine sur l'expression de marqueurs cutanés dermiques associés à la fermeté cutanée.

La présente invention vise encore l'utilisation cosmétique par voie orale et/ou parentérale à titre d'agent stimulant l'expression de marqueurs cutanés notamment choisis parmi la vimentine, la décorine, la fibroméduline, la biglycane et l'hyaluronsynthase.

Tous ces tests mettent particulièrement en exergue que la glucosamine permet d'agir sur le métabolisme cellulaire et les propriétés biomécaniques de la peau et tout particulièrement sur la fermeté cutanée.

L'invention vise donc également un procédé cosmétique pour favoriser le rétablissement de l'homéostasie de l'épiderme et/ou pour limiter la dégradation des constituants matriciels du derme et/ou pour réduire le niveau d'expression de la MMP3 et/ou pour stimuler le cytosquelette des fibroblastes et/ou pour améliorer les propriétés contractiles des fibroblastes et/ou pour augmenter l'expression de marqueurs cutanés notamment choisis parmi la vimentine, la décorine, la fibroméduline, le biglycane et l'hyaluronsynthase et ainsi réduire la cellulite ainsi que les aspects visuels associés et/ou maintenir et/ou restaurer la fermeté cutanée induite par la ménopause, ledit procédé comprenant l'administration par voie orale d'une composition contenant de la glucosamine éventuellement associée avec un composé polyphénol.

Selon un mode de réalisation particulier, l'invention est plus particulièrement relative à l'utilisation cosmétique par voie orale de la glucosamine à titre d'actif destiné à maintenir et/ou restaurer la fermeté cutanée par action de synthèse et/ou de protection des glucosaminoglycannes et des protéoglycannes, et plus particulièrement des Small-Leucine-Rich Proteoglycans (SLRP).

La présente invention décrit en outre l'utilisation cosmétique par voie orale et/ou parentérale de la glucosamine à titre d'actif destiné à maintenir et/ou restaurer les propriétés biomécaniques de la peau, notamment à maintenir et/ou restaurer les propriétés d'extensibilité, de tonicité, de fermeté, de souplesse, de densité et/ou d'élasticité de la peau.

Ces désordres liés à une perte des propriétés d'extensibilité, de tonicité, de fermeté, de souplesse et/ou d'élasticité de la peau, peuvent notamment être induits par le chronovieillissement, le vieillissement extrinsèque en particulier le photovieillissement ou le vieillissement hormonal notamment des peaux matures des femmes pré ou post ménopausées.

L'utilisation de la glucosamine par voie orale, selon la présente invention et comme décrit dans les revendications , est donc adaptée à la prévention et/ou au traitement des désordres cutanés concernant la perte de fermeté cutanée, induite par la ménopause.

Selon un mode de réalisation de la présente invention, la glucosamine peut être associée à au moins un composé polyphénol.

### COMPOSE POLYPHENOL

Les composés polyphénols regroupent une grande famille de composés très largement répandus dans le règne végétal. On les trouve ainsi notamment dans les plantes, depuis les racines jusqu'aux fruits. Parmi les classes de polyphénols, on peut notamment citer les flavonoïdes, les proantocyanidines, les lignanes, les lignines, les stilbènes, les coumarines. Ainsi, le composé polyphénol mis en oeuvre dans le cadre de la présente invention peut se présenter sous une forme isolée ou sous toutes les formes citées ci-après.

Dans le cadre de la présente invention, le composé polyphénol peut notamment dériver d'extraits végétaux choisis parmi les extraits de thé vert, de raisin tels que le *Vitis Vinifera,* de pin et notamment d'écorce de pin, de pomme, de myrtille, de houblon, de goyave, de cacao, de bois tels que le châtaignier, le chêne, le marronnier d'Inde, le noisetier.

Le terme « composé polyphénol » dans le cadre de la présente invention s'étend donc également à l'extrait végétal lui-même, riche en ces composés polyphénols.

Les flavonoïdes représentent le principal groupe de polyphénols.

Les polyphénols catéchiques constituent, quant à eux, un sous groupe des flavonoïdes, qui comprennent également les flavanones, les flavones et anthocyanines, et les flavonols.

Le composé polyphénol présent dans la composition à titre de premier objet de la présente invention et dans le complément alimentaire à titre de deuxième objet de la présente invention, est issu d'écorce de pin.

Un tel composé polyphénol issu d'écorce de pin présente avantageusement une teneur en trimères phénoliques pouvant aller de 5 à 25 % en poids, de préférence de 10 à 20 % en poids par rapport au poids total du composé polyphénol. De même, il présente avantageusement une teneur en dimères polyphénoliques pouvant aller de 5 à 25 % en poids, de préférence de 10 à 20 % en poids par rapport au poids total du composé polyphénol.

Le composé polyphénol issu d'écorce de pin contient aussi avantageusement de 2 à 15 % en poids, par exemple de 5 à 10 % en poids, d'acides phénoliques de type acide ferrulique, acide p-coumarique, acide caféique et acide protocatéchique, par rapport au poids total du composé polyphénol.

Ainsi, le composé polyphénol, particulièrement avantageux pour la mise en oeuvre de l'invention, peut présenter les caractéristiques suivantes :

| **Analyse/critère** | **Spécification** |
|---|---|
| Perte à la dessiccation | ≤ 5,0 % |
| Cendres sulfuriques | ≤ 0,4 % |
| Insolubles dans l'eau (solution à 1 %, T = 37 °C) | ≤ 5,0 % |
| Insolubles dans THF (solution à 1 %, T = 20 °C | ≤ 1,0 % |
| pH (solution aqueuse à 4 %, T = 20 °C) | 2,5 - 4,5 |
| Trimères polyphénoliques | 10-20 % |
| Dimères polyphénoliques | 10-20 % |
| Taxifoliol + taxifoliol glucoside | > 3 % |
| Teneurs en acides phénoliques ⁽¹⁾ | 2 - 15 % |

| | |
|---|---|
| ⁽¹⁾ acides ferrulique + p-coumarique + protocatéchique + caféique. | |

Aussi, selon une variante de réalisation de l'invention dans ses premier et deuxième objets, le composé polyphénol issu d'écorce de pin provient d'un extrait de pin maritime. Un tel extrait de pin maritime est notamment décrit dans l'article « A review of the French Maritime Pine Bark Extract (PYCNOGENOL®), a herbal medication with a diverse clinical pharmacology », P. ROHDEWALD, Internationl Journal of Clinical Pharmacology and Therapeutics, Vol. 40-No 4/2002(158-168),

Selon un mode de réalisation privilégié de l'invention, en particulier dans le cadre de la mise en oeuvre du troisième objet de l'invention, le composé polyphénol est un polyphénol catéchique tel que défini ci-après et selon un mode de réalisation tout particulièrement préféré, il est un composé polyphénol issu d'écorce de pin.

Le sous groupe des polyphénols catéchiques comprend un ensemble de composés classiquement isolés de plantes telles que le cacao, le thé, la vigne et ses dérivés, le pin (*Pinus maritima*), le cachou, certains fruits et présentant un degré de polymérisation variable.

L'unité de base, aussi appelée catéchine ou catéchol est le penta hydroxy-3,5,7,3',4' dihydro-2,3 phényl-2 chromène, qui peut être sous forme cis ou trans; l'épicatéchine est son isomère, et peut également être présent sous forme cis ou trans.

Les polyphénols catéchiques englobent aussi bien les divers isomères des unités de base (monomères), que des oligomères (ou proanthocyanidols) ou des polymères (tannins).

Plus particulièrement, les polyphénols catéchiques utiles selon l'invention sont choisis dans le groupe comprenant : catéchine, épicatéchine, gallocatéchine, épigallocatéchine et leurs sels, leurs esters et/ou leurs dérivés sous forme monomères ou oligomères.

Lorsque des oligomères sont utilisés, ils comportent avantageusement de 2 à 14 unités de base, notamment de 2 à 10.

De préférence leur degré de polymérisation est inférieur ou égal à 5.

On utilise en particulier des composés généralement appelés proanthocyanidoles ou procyanidols, encore appelés précurseurs d'anthocyanines ou oligomères procyanidoliques (OPC). Ces oligomères seront dégradés, pour une partie, après absorption par voie orale pour libérer les monomères.

Ces polyphénols peuvent être conjugués avec des sucres comme par exemple glucose, galactose, rhamnose, acide galacturonique. Par polyphénols catéchiques utiles selon l'invention on entend notamment dans le présent texte des mélanges en toutes proportions de monomères et des différents oligomères comportant de 2 à 14 unités, tels que définis précédemment.

Parmi les dimères largement répandus de la famille des procyanidols ou oligomères procyanidoliques et dont la mise en oeuvre est particulièrement avantageuse dans le cadre de la présente invention, on peut citer la procyanidine B1, la procyanidène B2, la procyanidine B3 ou encore la procyanidine B6 ou B7.

Les procyanidines B1, B2, B3 sont présentes notamment dans des extraits végétaux de cacao, de pomme, de myrtille, de marronnier d'Inde, de houblon, de goyave et de noisetier. Ainsi, on privilégie outre l'extrait d'écorce de pin, également l'utilisation d'un de ces extraits végétaux dans le cadre de la mise en oeuvre notamment du troisième objet de la présente invention, à savoir l'utilisation cosmétique de l'association de glucosamine et d'au moins un composé polyphénol à titre de mélange d'actifs destiné à maintenir et/ou restaurer les propriétés biomécaniques de la peau.

La composition selon l'invention apporte de préférence le composé polyphénol à une dose journalière allant de 1 à 1000 mg/jour, de préférence de 10 à 150 mg/jour, et de façon encore plus préférée de 30 à 100 mg/jour.

La composition selon l'invention comprend de préférence le composé polyphénol dans une teneur allant de 0,0001 à 50 % en poids, de préférence de 0,05 à 10 % en poids, et de façon encore plus préférée de 0,5 à 2 % en poids par rapport au poids total de la composition.

L'utilisation cosmétique par voie orale et/ou parentérale de l'association de glucosamine et d'au moins un composé polyphénol à titre de mélange d'actifs, permet de à maintenir et/ou à restaurer les propriétés biomécaniques de la peau.

L'association de glucosamine et d'au moins un composé polyphénol est en particulier destinée à maintenir et/ou à restaurer les propriétés d'extensibilité, de tonicité, de fermeté, de souplesse, de densité et/ou d'élasticité de la peau.

Comme l'illustrent les exemples 6 et 7 qui suivent, les inventeurs ont démontré qu'une composition contenant de la glucosamine et un composé polyphénol issu d'écorce de pin, pouvait agir d'une part favorablement sur la matrice dermique, par le biais d'une activation accrue du métabolisme cellulaire et une action ciblée sur le cytosquelette des fibroblastes, et d'autre part sur l'expression de marqueurs cutanés dermiques associés aux propriétés biomécaniques de la peau et en particulier à la fermeté cutanée.

Par conséquent, une composition contenant de la glucosamine et un composé polyphénol issu d'écorce de pin, est particulièrement adaptée à la prévention et/ou au traitement des désordres liés à une perte des propriétés d'extensibilité, de tonicité, de fermeté, de souplesse et/ou d'élasticité de la peau, en particulier induits par le chronovieillissement, notamment des peaux matures des femmes pré ou post-ménopausées, mais encore induits par le photovieillissement.

Cette composition est donc également adaptée à la prévention et/ou au traitement des désordres cutanés induits par la ménopause.

Ainsi, la présente invention concerne également l'utilisation cosmétique d'une composition contenant de la glucosamine et un composé polyphénol issu d'écorce de pin, conforme à l'invention destinée à la prévention et/ou au traitement de la perte de la fermeté cutanée induite par la ménopause.

Les compositions décrites peuvent être des compositions cosmétiques, dermatologiques ou pharmaceutiques.

Au sens de la présente invention, une composition cosmétique désigne une composition apte à produire un effet au niveau de la peau sur le plan esthétique et du confort, ou encore à visée beauté, par exemple en vue de la protéger, de la maintenir en bon état, d'en modifier l'aspect, et notamment de l'embellir. Elle peut se présenter sous la forme d'un produit nutritionnel.

Les compositions administrées par voie orale peuvent se présenter sous toutes les formes galéniques normalement utilisées selon le mode d'administration concerné.

Dans le cas de la voie orale, une composition telle que décrite peut être mise en oeuvre dans une formulation de type complément alimentaire ou aliment fonctionnel ou encore de type composition pharmaceutique.

Une telle composition peut notamment se présenter sous forme de capsules molles ou de gélules, de gélules banderolées, de gels, d'émulsions sèches ou liquides, de comprimés, de poudres à diluer ou d'ampoules buvables ou toute autre forme connue de l'homme du métier.

La composition peut éventuellement contenir des excipients de formulation appropriés tels que colorant, édulcorant, agent de charge, liant, conservateur, etc.

Selon un mode de réalisation privilégié, les principes actifs peuvent être incorporés dans des matrices alimentaires en vue de produire des aliments fonctionnels tels que des barres alimentaire, des aliments enrichis tels que des huiles, des margarines, des poudres compactées, des fibres ou encore sous la forme d'émulsion dans les boissons.

La composition peut en outre contenir des composés tels que des anti-oxydants, des vitamines, des minéraux autorisés en Europe dans les compléments alimentaires tels que décrits dans la Directive CE 2002/46.

Dans le cas de l'administration par voie parentérale, une composition conforme à la présente invention peut être sous forme d'une solution injectable ou d'un patch ou système de délivrance transdermique.

Selon un mode de réalisation avantageux de l'invention, la composition mettant en oeuvre la glucosamine seule ou éventuellement associée à au moins un composé polyphénol comprend en outre au moins un actif nutritionnel anti-âge, un actif nutritionnel photoprotection, un actif nutritionnel ménopause et/ou un actif nutritionnel minceur.

Parmi les actifs nutritionnels anti-âge, on peut notamment citer les antioxydants alimentaires, les nutriments aux propriétés anti-radicalaires et les cofacteurs des enzymes endogènes antioxydants : les vitamines A, C, E, les caroténoïdes tels que le lycopène, les xantophyles, les isoflavones, certains minéraux tels que le zinc, le cuivre, le magnésium, le sélénium, l'acide lipoïque, le co-enzyme Q10, la Superoxyde dismutase (SOD) ou encore la taurine. Parmi les actifs anti-âges, on peut notamment citer les fractions insaponifiables extraits de lipides d'origine végétale, aloe vera, collagène marin natif ou hydrolysé, huiles végétales ou marines riches en acides gras oméga-3, en oméga-6 (y compris l'acide gamma-linolénique), ...

Parmi les actifs nutritionnels photoprotection, on peut notamment citer : les antioxydants et les antiradicalaires : les vitamines A, C, E, caroténoïdes, xantophyles, certains minéraux tels que le zinc, le cuivre, le magnésium, le sélénium, la co-enzyme Q10, la superoxyde dismultase (SOD), les probiotiques.

On peut citer aussi les ingrédients nutritionnels présentant des propriétés d'hydratation ou encore immunomodulatrices : probiotiques, extrait de polypodium leucotomos, huiles végétales ou marines riches en acides gras Ω-3, en Ω-6, y compris l'acide gamma-linolénique.

Dans le cadre de la présente invention, lorsque la glucosamine est associée à au moins un composé polyphénol issu d'écorce de pin, la composition comprenant cette association peut comprendre en outre d'autres composés polyphénols que le composé polyphénol issu d'écorce de pin, à titre additionnel.

Parmi les actifs nutritionnels actifs sur les signes cliniques de la ménopause (par exemple bouffées de chaleur, ...), on peut notamment citer les isoflavones, les lignanes, la DHEA, les extraits de yam, de sauge, de houblon, le calcium, le magnésium, les hydrolysats de protéines, les huiles végétales ou marines riches en acides gras oméga-3.

Parmi les nutritionnels ingrédients mis en oeuvre dans le domaine de la minceur, on peut notamment citer : thé vert notamment sous forme d'extrait, thé blanc, thé noir, thé gris, rooïbos (encore appelé thé rouge), maté, marron d'inde, kola, caféine, théobromine, synéphrine, bromelaïne, éphédra, citrus aurantium, calcium, hoodia, garcinia, chitosan, fibres végétales (cactus, pommes, ananas, ...), fenouil, cassis, reine des près, radis noir.

L'extrait de thé vert est particulièrement intéressant de part sa teneur naturelle en flavonoïdes et plus particulièrement en catéchines et gallate d'épigallocatéchine (EGCG), conférant notamment des propriétés antioxydantes.

Selon un mode de réalisation particulier, l'invention concerne une composition pour administration orale comprenant l'association de glucosamine, d'au moins un composé polyphénol issu d'écorce de pin et d'un extrait de thé vert. Selon un mode de réalisation encore plus particulier, cette même composition comprend en outre du calcium, par exemple sous forme de carbonate, de calcium d'origine marine, de lactate ou de citrate, de calcium.

Selon un autre mode de réalisation, la présente invention concerne également l'utilisation cosmétique par voie orale de l'association de glucosamine, d'au moins un composé polyphénol ainsi qu'optionnellement de calcium, à titre de mélange d'actifs.

Selon ces deux modes de réalisation, le thé vert peut être présent dans la composition dans une teneur variant de 50 mg à 3 g, notamment de 300 à 800 mg, cette composition étant préférentiellement administrée à raison d'une dose par jour.

Toujours selon ces deux modes de réalisation, le calcium peut être présent dans la composition dans une teneur variant de 300 mg à 2 g, préférentiellement de 300 mg à 1 g à raison préférentiellement d'une dose par jour.

Le complément alimentaire conforme à la présente invention comprenant de la glucosamine dans une première composition et au moins un composé polyphénol issu d'écorce de pin dans une deuxième composition, comme kit ou produit de combinaison pour une utilisation simultanée, séparée ou étalée dans le temps, peut être formulé de telle façon que les deux compositions sont sous les mêmes formes ou sous des formes différentes, par exemple choisies parmi celles citées plus haut. Un tel kit peut notamment être présenté dans un seul et même emballage ou dans deux emballages distincts, un pour chaque composition.

Les exemples 1, 4, 5 et 7 qui suivent illustrent la présente invention. Les exemples 2, 3 et 6 sont des exemples de référence. Les exemples 8-11 sont des exemplifications de formulations de glucosamine pouvant être utilisés selon la présente invention.

### Exemple 1 : effet de la glucosamine sur la synthèse du collagène et l'expression du CD44-modèle in vitro d'explants de peau

Le but de cette étude a été de mettre en évidence l'effet de la glucosamine (sous forme sulfatée) sur un marqueur épidermique (le CD44), potentiellement impliqué dans la pathogénèse de l'atrophie cutanée, manifestation majeure du vieillissement cutané, et sur la synthèse de collagène.

Pour cela, une méthode de culture a été utilisée permettant la mise en survie de peau humaine dans des conditions métaboliques proches de l'in vivo (sujets d'âge compris entre 50 et 60 ans). La glucosamine en solution aqueuse a été ajoutée dans le milieu de culture, à la concentration plasmatique (5 µM), permettant ainsi de simuler une administration par voie orale et/ou parentérale.

Au niveau de l'épithélium, a été examiné le récepteur épidermique de l'acide hyaluronique (anticorps anti-CD44). Au niveau dermique, la néosynthèse du collagène par les fibroblastes (dosage biochimique) a été évaluée.

### MATERIEL ET METHODES

### • Maintien en survie de peau humaine

Des fragments de peau ont été obtenus après chirurgie plastique (plasties mammaires ou abdominales) chez des femmes ménopausées (8 donneurs différents de sujets d'âge compris entre 50 et 60 ans et de sexe féminin). Les fragments sont déposés dans des inserts eux-mêmes disposés en suspension au-dessus de puits de culture. Du milieu de culture est ajouté dans le fond des puits, un passage s'effectuant par diffusion lente entre les deux compartiments par l'intermédiaire d'une membrane poreuse (12 µm).

De J0 à J10, la glucosamine a été ajoutée dans le milieu de culture des fragments de peaux, tous les jours. Pour l'analyse du CD44, une série de peaux a été prélevée à J4. Une seconde série a été prélevée à J10 pour le dosage de la synthèse de collagène.

### • Analyses

### Mise en évidence immunohistochimique du récepteur de l'acide hyaluronique au niveau de l'épithélium (CD44)

Il est possible de mettre en évidence une glycoprotéine transmembranaire de 80-95 kD, le CD 44 (H-CAM, Novocastra, dilution au 1/300), récepteur de l'acide hyaluronique au niveau de l'épithélium. L'immunodétection a été réalisée à l'aide du kit CSA (DAKO) et révélée en rouge par l'AEC (3 amino- 9 éthyl - carbazole).

Des scores semi-quantitatifs ont permis de préciser l'intensité de l'immunomarquage (scores 0 à 4, de négatif à intense). La topographie a également précisée à l'aide de scores :
- score de 0 : pas de marquage
- score 1 : marquage de la couche basale,
- score 2 : marquage de la couche basale et du tiers de l'épithélium,
- score 3 : marquage de la couche basale et des deux tiers du corps muqueux
- score 4 : marquage de l'ensemble de l'épithélium.

### Dosage biochimique du collagène

Après les 10 jours de mise en survie, les fragments de peaux sont digérés enzymatiquement pendant une nuit à +4 °C dans une solution d'acide acétique à 0,5M contenant de la pepsine. Cette méthode permet de récupérer le collagène nouvellement synthétisé. Après broyage à l'aide d'un potter, la quantité de collagène (µg/ml) est évaluée par une méthode de dosage spectrocolorimétrique à 540 nm : le collagène acido-soluble est détecté après fixation spécifique du colorant rouge sirius (Sircol Collagen Assay, Interchim).

Pour comparer les différents résultats, la quantité de collagène est rapportée à la quantité de protéines totales de l'échantillon. Le dosage de la concentration en protéines a été réalisé spectrophotométriquement à 562 nm (BCA assay, Pierce). Le résultat final est exprimé en µg de collagène / mg de protéine.

### Résultats

### • Mise en évidence immunohistochimique du récepteur de l'acide hyaluronique au niveau de l'épithélium (CD44)

Les résultats sont exprimés dans le Tableau I. En présence de glucosamine, on constate une augmentation de façon significative de l'intensité du marquage du CD44 avec un score de 3,4 versus 2,7 pour la peau témoin (p = 0,02).

**Tableau 1 : analyse immunohistochimique du récepteur épithélial de l'acide hyaluronique, CD44 (scores semi-quantitatifs moyens de topographie et d'intensité du marquage)**

| | **Score intensité** | **Score topographie** |
|---|---|---|
| Peau témoin | 2,7 ± 1,1 | 3,4 ± 0,7 |
| Peau + glucosamine (5 µM) | 3,4 ± 0,7 | 3,7 ± 0,5 |
| | * p = 0,02 | p = 0,054 |

| | | |
|---|---|---|
| * : différence statistiquement significative par rapport à la peau témoin (test apparié de Student, p < 0,05) | | |

### • Dosage biochimique du collagène

La synthèse de collagène est significativement augmentée en présence de glucosamine : 282 µg/ml (p = 0,031) en comparaison avec la peau témoin où le taux est de 177,5 µg/ml.

### Conclusion

Dans les conditions de test, la glucosamine administrée à 5 µM stimule significativement i) la synthèse de collagène par les fibroblastes dermiques ainsi que 2) l'expression du récepteur de l'acide hyaluronique CD44. La glucosamine apparaît donc comme un activateur du métabolisme du fibroblaste et un agent promoteur du rétablissement de l'homéostasie épidermique, paramètres généralement altérés avec l'âge.

### Exemple 2 : évaluation de l'activité anti-dégradative de la glucosamine

L'objectif de ces essais est d'évaluer l'effet du sulfate de glucosamine sur l'activité anti-dégradative des macromolécules du derme (activité anti-MMP) et sur leurs propriétés contractiles (action sur le cytosquelette). L'effet sur les propriétés mécaniques des fibroblastes est évalué par la mesure de la vitesse de rétraction d'un gel tri-dimensionnel de collagène.

### MATERIEL ET METHODES

### Cultures de fibroblastes

Des fibroblastes humains, récoltés par croissance à partir d'explants de peau d'un donneur normal jeune, sont cultivés en routine dans le DMEM contenant 10 % de sérum bovin foetal (FCS), acide ascorbique (50 µg/ml), pénicilline-streptomycine (100 U/ml) référencé dans la suite du rapport comme DMEM-FCS. Ils sont amplifiés en culture par trypsinisation de cultures sub-confluentes et division des boites 1 en 3. Les cultures sont utilisées jusqu'au passage 14. Elles sont testées régulièrement pour l'absence de mycoplasme.

### Préparations des solutions et stérilisation

Le sulfate de glucosamine est fourni sous la forme de (C₆H₁₄N₀₅)₂ S04.2KCl (titre en Glucosamine.SO₄ : 74 %) de marque Bioiberica, code F0379, Batch 4/0001. Une solution stock à 13.6mg/ml d'eau distillée est stérilisée par filtration sur Acrodisc et conservée à -20 °C.

### 2.1. Cytotoxicité

Les fibroblastes sont ensemencés dans des multiwells 24 puits à 25 ou 50x 10³ cellules/puit dans le DMEM-FCS. Après 6h d'attachement, 20 µl de dilutions progressives des actifs à tester sont ajoutés aux cultures. Après 24h de contact, le milieu et les actifs sont renouvelés. Après 48h de culture, le milieu est éliminé, les couches cellulaires sont rincées à deux reprises par le liquide physiologique. Le nombre de cellules est déterminé par mesure de DNA par technique fluorimétrique à l'aide du réactif bis-benzimide dans un fluorimètre « Gemini ». Les cultures sont réalisées en triplicate et les mesures sur chaque culture en duplicate. Les cultures contrôles reçoivent le solvant seul.

### Sulfate de glucosamine

La toxicité du sulfate de glucosamine est testée à 0,5 ; 1 ; 5 ; 10 ; 20 et 40x la concentration plasmatique, c'est-à-dire 3,4 ; 6,8 ; 34 ; 68 ; 136 ; 272 µg/ml de milieu de culture.

| **[x** × **Concentration plasmatique]** | **µg/ml** | **N** | **DNA (µg/ml)** |
|---|---|---|---|
| 0 | 0 | 6 | 2,37 ± 0,33 |
| 0,5x | 3,4 | 3 | 2,81 ± 0,41 |
| 1x | 6,8 | 3 | 2,56 ± 0,37 |
| 5x | 34 | 3 | 2,89 ± 0,23 |
| 10x | 68 | 3 | 2,61 ± 0,26 |
| 20x | 136 | 3 | 2,52 ± 0,18 |
| 40x | 272 | 3 | 2,50 ± 0,32 |

| | | | |
|---|---|---|---|
| x = concentration plasmatique | | | |

Aucun effet cytotoxique n'est observé pour le sulfate de glucosamine même aux concentrations représentant 40x la concentration plasmatique. Un accroissement du nombre de cellules (p=0.04, test de Student) est observé à la concentration de 34 µg/ml indiquant un léger effet stimulant sur la vitesse de prolifération des fibroblastes.

### 2.2. Effet du sulfate de glucosamine l'activité des MMPs (ARNm - modèle de fibroblastes dermiques humains en monocouche sur plastic)

### 1. Préparation des cultures et purification de l'ARN total

Les fibroblastes sont ensemencés dans le DMEM-FCS à raison de 2.10⁵ cellules/disque de 6 cm. Après 18h d'attachement, le milieu de culture est renouvelé par le DMEM-FCS contenant :
a) sulfate de glucosamine seul

| | |
|---|---|
| - [concentration plasmatique] | 2x (n=2) |
| | 5x (n=2) |
| | 10x (n=2) |
| - contrôle | (n =3) |

### 2. Mesure des ARN de référence (28S et GAPDH) et des ARN messagers spécifiques par RT-PCR

La quantité totale d'ARN présente dans la solution qui servira à réaliser les mesures des ARNm spécifiques est déterminée par mesure de l'ARN ribosomial 28S. La solution d'ARN est aliquotée et congelée à -80 °C jusqu'aux dosages par RT-PCR des ARNm spécifiques. Les résultats des ARNm sont exprimés en unités arbitraires (UA) par unité de 28S. Les mesures sont réalisées en triplicate sur deux ou trois cultures séparées.
• **MMP3 :** stromélysine 1 responsable de la dégradation des protéoglycannes et d'autres constituants de la matrice extracellulaire et activateur de la MMP1.

| | **[conc plasma]** | UA/ 28 S |
|---|---|---|
| | **0** | 1745 ± 31 |
| **glucosamine** | **2 x** | 1261 ± 120 |
| | **5 x** | 1162 ± 22 |
| | **10 x** | 1060 ± 375 |

Une réduction significative du niveau de l'ARNm de la MMP3 est induite par le sulfate de glucosamine (p<0,005).

**Conclusion** : la glucosamine réduit le niveau d'expression de la MMP3. Ceci indique une activité anti-dégradative des constituants matriciels du derme, en particulier les protéoglycannes et glycosaminoglycanes. Ces résultats démontrent que la glucosamine est un agent capable de protéger les macromolécules du derme dont l'altération contribue à la perte de fermeté cutanée ou encore à l'aggravation des désordres cutanés induits par la cellulite.

### Exemple 3 : Effet du sulfate de glucosamine sur les propriétés contractiles des fibroblastes

Une solution de collagène natif purifié stérile est mélangée à une suspension de fibroblastes, du milieu DMEM-FCS et les produits à tester dans des disques bactériologiques. Le mélange est porté à 37 °C ce qui résulte en une polymération du collagène et la formation d'un gel tri-dimensionnel de collagène emprisonnant les fibroblastes et flottant dans le mileu de culture. Les cellules s'attachent aux fibres de collagène et sous l'activité contractile du cytosquelette des fibroblastes, le gel est progressivement rétracté. Ce gel est communément nommé « Retracting Collagen Gel » ou RCG. L'article Charles A. Lambert et al. « An Interleukin-1 loop is induced in human skin fibroblasts upon stress relaxation in a three-dimensional collagen gel but is not involved in the up-regulation of Matrix Metalloproteinase 1", The Journal of Biological Chemistry, Vol. 273, No. 36, pp. 23143-23149, 1998, décrit l'utilisation d'un tel gel de collagène.
**a-** Une première série de gels contenant 25 000 fibroblastes a été réalisée. La glucosamine a été testée à la concentration plasmatique en triplicate. Les cultures contrôles reçoivent le solvant seul. Les mesures de diamètre du gel montrent une réduction du diamètre de 21 mm en 18 heures pour les contrôles et de 23 mm sur le même laps de temps pour la glucosamine. Cette légère différence s'atténue en fonction du temps, jusqu'à 50 heures de culture.
**b-** Ces résultats ont incité les inventeurs à refaire le même type d'expérience, avec le sulfate de glucosamine seul à 2x, 5x et 10x la concentration plasmatique en réduisant légèrement le nombre de fibroblastes (20 000) pour ralentir le processus. La vitesse de rétraction est bien ralentie puisqu'une réduction du diamètre du gel de 19 mm est observée en 46 h. De nouveau, une rétraction légèrement supérieure (22 mm en 46 h) est observée avec les trois concentrations testées de glucosamine.

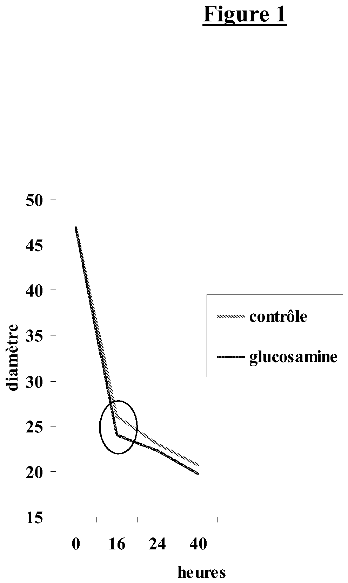

**Conclusion** : La figure 1 illustre la rétraction et donc la stimulation des propriétés contractiles des fibroblastes qui est observée en présence de sulfate de glucosamine Ces résultats démontrent l'activité de la glucosamine sur la stimulation du cytosquelette et du métabolisme du fibroblaste et qu'elle est de ce fait, un agent capable d'agir positivement sur la fermeté cutanée ainsi que sur les désordres cutanés induits par la cellulite.

### Exemple 4 : Effet du sulfate de glucosamine sur l'expression de marqueurs cutanés dermiques associés a la fermeté cutanée.

### 1. Protocole

Etude monocentrique en double aveugle *versus* placebo, randomisée sous contraintes. Quinze femmes, âgées de 50 à 65 ans, ont été supplémentées pendant 8 semaines soit avec un placebo (n=7) soit avec du sulfate de glucosamine (n=8) équivalente à 250 mg de glucosamine (GLU).

Une biopsie cutanée de 3 mm de diamètre (face interne du bras) a été réalisée avant le début (T0) et au terme des 8 semaines de supplémentation (T8).
L'ARN total contenu dans les prélèvements cutanés a été extrait et purifié au moyen d'un kit « Ambion Ribo Pure kit ».

L'analyse des marqueurs dermiques spécifiques a été réalisée par RT-PCR comme précédemment décrit (Nusgens et al, JID, 116 : 853-859, 2001). Tous les résultats sont exprimés en unités arbitraires rapportés à la quantité d'ARNr 28S.

A T0, l'homogénéité des valeurs de chaque ARNm, entre le groupe placebo et le groupe GLUT, a été vérifiée (test ANOVA et test de comparaison multiple de Tukey).

Les effets de la supplémentation (placebo ou GLU) ont ensuite été analysés en comparant, pour chaque volontaire, l'expression individuelle des ARNm à T8 et à T0. Un ratio T8/T0 a ainsi été calculé. Les moyennes de chaque ratio des deux groupes ont été comparées en utilisant un test de Student pour données appariées.

### 2. Résultats

Le tableau 2 résume les moyennes individuelles des taux d'ARNm avant (T0) et après 8 semaines de supplémentation (T8) dans le groupe placebo et dans le groupe GLU.

**Tableau 2**

| ARNm | PLACEBO (T8/T0) | GLU (T8/T0) |
|---|---|---|
| Vimentine | 1.27 ± 0.33 | 1.28 ± 0.33 |
| Decorine | 1.11 ± 0.22 | 1.11 ± 0.11 |
| Fibromoduline | 1.12 ± 0.16 | 1.10 ± 0.12 |
| Biglycan | 1.10 ± 0.18 | 1.15 ± 0.19 |
| Hyaluronsynthase | 1.51 ± 0.73 | 1.62 ± 1.42 |

On note que les résultats obtenus avec le placebo sont non significatifs tandis que les résultats obtenus avec la glucosamine sont significatifs.

### 3. Conclusion

La glucosamine (GLU) augmente de manière significative l'expression :
- de la vimentine, constituant du cytosquelette,
- de la décorine, de la fibromoduline et du byglican, appartenant à la famille des SLRPs (Small leucine Rich Proteins). Ces marqueurs sont impliqués dans l'organisation architecturale des structures de la peau,
- de la hyaluronsynthase, enzyme impliquée dans la synthèse de l'acide hyaluronique, dont les propriétés hydratantes sont connues de l'homme de métier.

Le placebo est sans effets sur ces différents marqueurs.

Ces résultats montrent que la glucosamine a un effet bénéfique sur l'expression de gènes spécifiques codant pour des macromolécules structurelles du derme. Cet effet s'avère particulièrement bénéfique pour maintenir et/ou pour restaurer la fermeté cutanée et prévenir et/ou traiter la cellulite, deux phénomènes caractérisés par une altération de l'architecture dermique.

### Exemple 5 : étude clinique exploratoire relative à la quantification des ARNm cibles induits par la prise du complément alimentaire A1

### Formule du complément alimentaire A1

Le complément alimentaire A1 est préférentiellement destiné aux personnes suivant un régime amaigrissant et qui sont confrontées à une perte de fermeté cutanée consécutive à la perte de poids. Le thé vert et le calcium sont des ingrédients respectivement reconnus comme adjuvant des régimes amaigrissants ou impliqué dans le métabolisme des lipides.
- Forme galénique : sachets
- Posologie : 1 sachet/jour

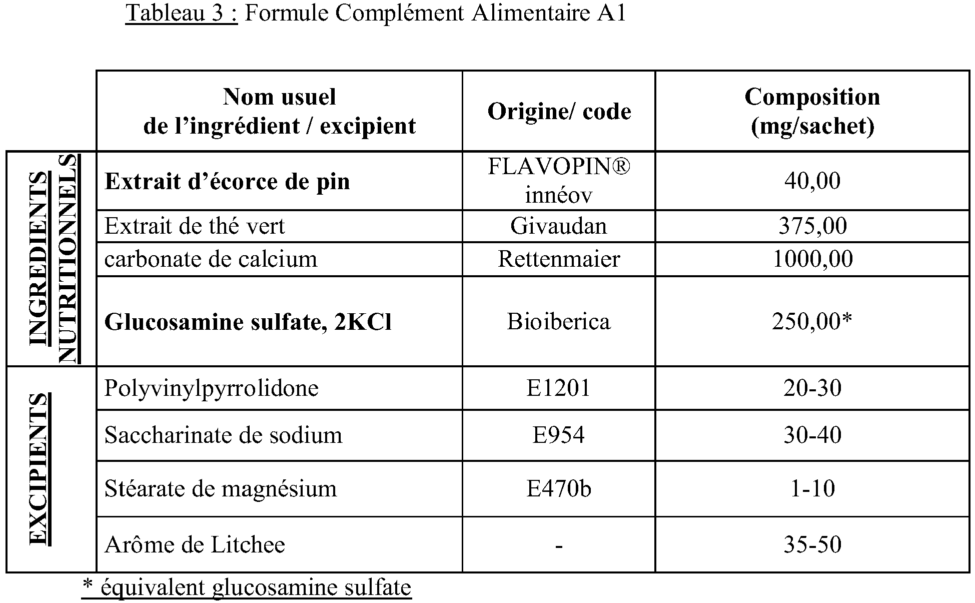

### Objectif de l'essai clinique

Evaluer l'effet du complément alimentaire A1 contre placebo, sur des biomarqueurs cutanés susceptibles d'être associés à un changement des propriétés biomécaniques de la peau après 2 mois de prise du complément alimentaire A1.

### Méthodologie

- Etude de 2 mois sur 16 femmes ménopausées depuis plus de deux ans (7 dans le groupe avec complément alimentaire A1 / 9 dans le groupe placebo), âgées de plus de 50 ans, ne suivant pas un traitement hormonal substitutif, et présentant un manque de fermeté cutanée au niveau de la face interne du bras.
- Réalisation de biopsies de 3 mm sur le bras à T0 et T2 mois pour extraction des ARNm totaux.
- Quantification d'ARNms spécifiques codant pour des protéines susceptibles d'être associées à un changement des propriétés biomécaniques de la peau selon le protocole suivant.

### Préparation des ARNm en vue de la RT-PCR

Les deux biopsies ont été poolées, broyées dans de l'azote liquide (Mikro Dismenbrator S, B. Braun Biotech International), puis l'ARN total a été extrait et purifié par ultracentrifugation sur gradient de chlorure de césium. La quantité d'ARN purifié a été évaluée en mesurant la DO (densité optique) à 260 nm (Nanodrop) et la qualité de l'ARN extrait a été validée par le calcul du ratio DO260/DO280. L'intégrité des ARN extraits a également été vérifiée en utilisant le BioAnalyzer de chez Agilent. Des solutions stocks d'ARN ont été préparées afin d'obtenir une concentration proche de 1,25 ng/µl.

### Evaluation de la quantité des biomarqueurs cutanés

La concentration en ARN a été normalisée par rapport à la quantité d'ARN ribosomal 28S. La technique de RT-PCR a été rendue quantitative en ajoutant dans chaque tube de réaction un standard interne de concentration connue, et constitué par une ARN synthétique, qui sera co-transcrit et co-amplifié en même temps que l'ARN recherché. Les échantillons correspondant au T0 et au T2 de chaque volontaire ont été analysés dans la même série, et ont fait l'objet d'un dépôt et d'une migration sur le même gel de polyacrylamide. L'analyse statistique a été réalisée au moyen d'un test de Student unilatéral pour échantillon apparié, en comparant dans chaque groupe les valeurs à T0 et à T2. Un ratio T2/T0 = 1,00 signifie que les deux valeurs sont comparables. Un ratio T2/T0 > 1,00 est le reflet d'une augmentation du transcript étudié. A l'inverse, un ratio T2/T0 < 1,00 est le reflet d'une diminution de l'expression de l'ARNm étudié.

### Résultats / Quantification d'ARNm cibles

**Tableau 4 : expression des gènes cibles après prise du complément alimentaire A1 (versus placebo)**

| | **T2/T0** | | **T2/T0 COMPLEMENT ALIMENTAIRE** | |
|---|---|---|---|---|
| | **Placebo (n=9)** | pairé t test | **A1 (n=7)** | pairé t test |
| **VIM** | *1,02* + *0,36* | NS | 1,43 + 0.47 | 0,025 |
| **BMP1** | 1,14±0,53 | NS | 1,55±0,67 | 0,03 |
| **DEC** | 1,33±0,58 | NS | 1,59±0,53 | 0,02 |
| **LUM** | 1,25±0,53 | NS | 1,69±0,55 | 0,004 |
| **FIBMOD** | 1,17±0,44 | NS | 1,93±0,77 | 0,03 |
| **ACTIN** | 0,89±0,32 | NS | 1,49±0,48 | 0,005 |

| | | | | |
|---|---|---|---|---|
| VIM : vimentine ; BMP1 : Bone Morphogenetic Protein 1 ; DEC : décorine ; LUM : lumican ; FIBMOD : fibromoduline ; ACTIN : actine. | | | | |

- La concentration en ARNm codant pour la vimentine est significativement augmentée (p=0,025) après prise du complément alimentaire A1 alors qu'il n'y pas de différence significative avec le placebo. La vimentine est une protéine faisant partie du cytosquelette des cellules du derme. La prise pendant 2 mois du complément alimentaire A1 entraîne une augmentation statistiquement significative de l'ARNm codant pour l'actine (p=0,0005), une autre protéine constitutive du cytosquelette. Le placebo est sans effet sur marqueur.

Le terme cytosquelette désigne le réseau de fibres intracellulaires dynamiques intervenant dans tous les mouvements de la cellule comme par exemple le transport intracellulaire des organites ou le maintien de la forme de la cellule. Les éléments du cytosquelette diminuent avec l'âge induisant un ralentissement du métabolisme de synthèse de la cellule.

Dans les cellules d'origine mesenchymenteuse, telles que les fibroblastes et les cellules endothéliales, la vimentine est un composé structural majeur des filaments intermédiaires. Ce réseau du cytosquelette est directement impliqué dans les fonctions mécaniques cellulaires. En particulier, une faible expression de la vimentine affecte les processus de cicatrisation. Les cellules déficitaires en vimentine présentent notamment une faible stabilité mécanique ainsi qu'une motilité et des propriétés contractiles réduites. De plus, la vimentine participe à l'organisation spatiale des complexes focaux, à l'organisation des microfilaments actine-dependants ainsi qu'aux interactions avec la matrice extracellulaire. Certaines de ces interactions sont directement affectées par le vieillissement cutané à l'instar de celles qui influent sur le contrôle de la migration, de la prolifération et de la contraction cellulaire ou encore sur le contrôle du phénotype métabolique.

L'actine est une protéine du cytosquelette qui participe à la formation des fibres d'actamyosine de stress et des polymères corticaux d'actine qui sont impliqués dans les fonctions mécaniques des cellules au sein du cytosquelette.

L'augmentation des ARNm codant pour l'actine et la vimentine indique donc que le complément alimentaire A1 agit favorablement sur le cytosquelette des fibroblastes. C'est le signe d'une activité accrue de la cellule.
- Le gène de la décorine est surexprimé de manière significative (p=0,02) suite à la prise du complément alimentaire A1 alors que l'expression de ce gène n'est pas modifiée suite à la prise du placebo. La décorine qui appartient à la famille des Small Leucine-Rich Proteoglycans (SLRP) est une petite protéine glycosylée (protéoglycanne). La décorine est présente dans l'ensemble du derme, mais absente de l'épiderme. Elle est synthétisée et sécrétée par les fibroblastes. En outre, après prise du complémentaire, on note aussi une augmentation significative des gènes codant pour d'autres Small Leucine-Rich Proteoglycans (SLRP), en particulier ceux du lumican (p=0,004) et de la fibromodulline (p=0,03). Ces marqueurs sont impliqués dans l'organisation architecturale des structures de la peau

Enfin, la prise du complément alimentaire A1 entraîne une augmentation statistiquement significative de l'expression de l'ARNm codant pour la BMP-1 (Bone Morphogenetic Protein) (p=0,03), alors que cet effet n'est pas retouvé avec le placebo. La BMP-1 est une enzyme qui clive les pro-collagènes I et III, formant ainsi des monomères matures capables de s'assembler au sein des fibrilles de collagène. Par l'action conjointe de l'enzyme BMP-1 et de l'ADAMTS-2, les propeptides de procollagène sont clivés pour permettre ensuite leur auto-polymérisation en fibres et faisceaux de fibres de collagène plus résistants au plan mécanique. La taille de ces fibres ainsi que leur régularité sont controlés par la décorine, le lumican et la fibromodulline. De plus, l'enzyme BMP-1 est directement impliqué dans la transformation du probiglycan en biglycan.

Les résultats sur les SLRP et la BMP-1 montrent que le complément alimentaire A1 semble avoir un effet stabilisateur sur la structure des fibres de collagène et contribue donc à améliorer la qualité de la matrice extracellulaire du derme.

Par ces deux résultats, d'une part l'action sur le cytosquelette révélée par l'étude de la vimentine et de l'actine et d'autre part l'action sur les fibres de collagène révélée par l'étude de la décorine et de la BMP-1, le complément alimentaire A1 présente une action globale et complémentaire sur le derme.

### Exemple 6 : étude clinique visant à évaluer l'effet du complément alimentaire A1 sur les propriétés cutanées chez la femme en surcharge pondérale suivant un régime amincissant

### Formule du complément alimentaire A1

Le complément alimentaire A1 est préférentiellement destiné aux personnes suivant un régime amaigrissant et qui sont confrontées à une perte de fermeté cutanée consécutive à la perte de poids. Le thé vert et le calcium sont des ingrédients respectivement reconnus comme adjuvant des régimes amaigrissants ou impliqué dans le métabolisme des lipides.
- Forme galénique : sachets
- Posologie : 1 sachet/jour

### Objectif de l'essai clinique

L'objectif principal de cette étude est d'évaluer l'effet du complément alimentaire A1 sur les propriétés cutanées au cours d'un régime amincissant chez des femmes à partir des données obtenues avec le score clinique et l'auto-évaluation des volontaires.

### Méthodologie

### • Phase clinique

- Etude de 3 mois sur 40 femmes (placebo n=19, A1 n=21), âgées de 25 à 45 ans, suivies par des nutritionnistes, en cabinet de ville, dans le cadre d'un régime amincissant. Cette étude a été réalisée versus placebo.
- Le score clinique est évalué par le dermatologue à T0 et T3 mois. Ce score clinique est la résultante de la somme des scores de laxité, flétrissure, aspect papyracé et ptose, évalués selon une échelle de 0 (nul) à 6 (important). Ce score reflète la tonicité de la peau.
- Les auto-évaluations ont été réalisées pour chaque patiente à T0 et à T3 mois à l'aide d'une échelle allant de 0 (pas) à 9 (très).

### Résultats

Score clinique : après 3 mois, la diminution du score clinique est statistiquement plus importante dans le groupe A1 que dans le groupe placebo (p=0,04). Il est a noté que les scores cliniques sont comparables à T0. Ce résultat est en faveur d'une amélioration des propriétés biomécaniques de la peau suite à la prise du complément alimentaire A1.

Auto-évaluation : le scorage réalisé par les volontaires met en évidence, après 12 semaines de prise de A1, des évolutions statistiquement significatives :
- Diminution de l'aspect peau d'orange au niveau de la cuisse, à la palpation (p=0,004) et après observation (p=0,01).
- Diminution de l'aspect peau flasque au niveau du bras (p=0,04) et de l'abdomen (p=0,04).
- Diminution de la douleur au pincement sur la cuisse (p=0,05).

Ces deux résultats mettent en évidence un effet bénéfique du complément alimentaire A1 sur les propriétés cutanées avec une amélioration de la tonicité cutanée et un lissage des capitons, signe caractéristique de la cellulite.

### Exemple 7 : Effet du sulfate de glucosamine + polyphénols (extrait d'écorce de pin maritime) sur l'expression de marqueurs cutanés dermiques associés aux propriétés biomécaniques de la peau.

### 1. Protocole

Etude monocentrique en double aveugle *versus* placebo, randomisée sous contraintes. Quinze femmes, âgées de 50 à 65 ans, ont été supplémentées pendant 8 semaines soit avec un placebo (n=7) soit avec un mélange de sulfate de glucosamine (équivalente à 250 mg de glucosamine) et d'extrait d'écorce de pin maritime (30 mg ; n=8, GLU 02).

Une biopsie cutanée de 3 mm de diamètre (face interne du bras) a été réalisée avant le début (T0) et au terme des 8 semaines de supplémentation (T8).

L'ARN total contenu dans les prélèvements cutanés a été extrait et purifié au moyen d'un kit « Ambion Ribo Pure kit ».

L'analyse des marqueurs dermiques spécifiques a été réalisée par RT-PCR comme précédemment décrit (Nusgens et al, JID, 116 : 853-859, 2001). Tous les résultats sont exprimés en unités arbitraires rapportés à la quantité d'ARNr 28S.

A T0, l'homogénéité des valeurs de chaque ARNm, entre le groupe placebo et le groupe GLUT, a été vérifiée (test ANOVA et test de comparaison multiple de Tukey).

Les effets de la supplémentation (placebo ou GLU 02) ont ensuite été analysés en comparant, pour chaque volontaire, l'expression individuelle des ARNm à T8 et à T0. Un ratio T8/T0 a ainsi été calculé. Les moyennes de chaque ratio des deux groupes ont été comparées en utilisant un test de Student pour données appariées.

### 2. Résultats

Le tableau 5 résume les moyennes individuelles des taux d'ARNm avant (T0) et après 8 semaines de supplémentation (T8) dans le groupe placebo et dans le groupe GLU 02.

**Tableau 5**

| ARNm | PLACEBO (T8/T0) | GLU (T8/T0) |
|---|---|---|
| Vimentine | 1.27 ± 0.33 | 1.36 ± 0.27 |
| Collagène A1 I | 1.48 ± 0.51 | 2.00 ± 0.94 |
| Collagène A1 III | 1.38 ± 0.41 | 1.94 ± 0.74 |
| Decorine | 1.11 ± 0.22 | 1.23 ± 0.16 |
| Lumican | 1.06 ± 0.23 | 1.12 ± 0.12 |
| Fibromoduline | 1.12 ± 0.16 | 1.21 ± 0.18 |
| Biglycan | 1.10 ± 0.18 | 1.22 ± 0.17 |
| Actine | 1.10 ± 0.15 | 1.16 ± 0.23 |

On note que les resultants obtenus avec le placebo sont non significatif tandis que les resultats obtenus avec l'association glucosamine + polyphénols sont significatifs.

### 3. Conclusion

L'association glucosamine + polyphénol augmente de manière significative l'expression :
- des collagènes de type I et III, constituants majeurs de l'architecture dermique.
- de la vimentine et de l'actine, constituants du cytosquelette,
- de la décorine, de la fibromoduline, du lumican et du byglican, tous membres de la famille des SLRPs (Small leucine Rich Proteins). Ces marqueurs sont impliqués dans l'organisation architecturale des structures de la peau.

Le placebo est sans effets sur ces différents marqueurs.

Ces résultats montrent que l'association de la glucosamine avec un composé polyphénol extrait d'écorce de pin maritime a un effet bénéfique sur l'expression de gènes spécifiques codant pour des macromolécules structurelles du derme. Cet effet s'avère particulièrement bénéfique pour maintenir et/ou restaurer les propriétés biomécaniques de la peau, y compris pour maintenir et/ou restaurer la fermeté cutanée et prévenir et/ou traiter la cellulite, deux phénomènes caractérisés par une altération de l'architecture dermique.

### Exemple 8 : Forme gélule (gélatine de poisson)

| Ingrédient/additif | Dosage (mg/capsule) |
|---|---|
| Sulfate de glucosamine, 2KCl | 200 |
| Cellulose microcristalline | 50 |

### Exemple 9 : Forme boisson

| Ingrédient/additif | Dosage (mg/75 ml) |
|---|---|
| Sulfate de glucosamine, 2KCl | 1000 |
| Eau | QSP100 |

### Exemple 10 : forme poudre à diluer (sachet)

| Ingrédient/additif | Dosage (mg/sachet) |
|---|---|
| Extrait d'écorce de pin | 20,0 |
| Glucosamine sulfate, 2 KCl | 170,0 |
| Extrait de thé vert | 187,5 |
| Calcium carbonate | 500,0 |
| Amidon modifié | 500 |
| Silice colloïdale | 32,0 |
| Aspartame | 35,0 |
| Allura red | 11,2 |

La posologie est de 2 sachets par jour.

### Exemple 11 : forme capsule (gélatine de poisson)

| Ingrédient/additif | Dosage (mg/capsule) |
|---|---|
| Extrait d'écorce de pin | 10,0 |
| Glucosamine sulfate, 2 KCl | 85,0 |
| Extrait de thé vert | 93,7 |
| Calcium carbonate | 250,0 |
| Stéarate de magnésium | 10,0 |
| Cellulose microcristalline | 46,2 |
| Sodium carboxyl methyl cellulose | 5,0 |

La posologie est de 2 à 4 gélules par jour.

## Revendications

1. Utilisation cosmétique par voie orale de la glucosamine comprenant toutes ses formes salifiées, acétylées et/ou polymériques dans la prévention et/ou le traitement de la perte de fermeté cutanée induite par la ménopause.

2. Utilisation selon la revendication 1, **caractérisée en ce que** la glucosamine agit en tant qu'activateur de la synthèse du collagène et/ou en tant qu'agent promoteur du rétablissement de l'homéostasie épidermique et/ou à titre d'agent d'inhibition de l'expression de la MMP3 et/ou en tant qu'agent favorisant la contractilité des fibroblastes et/ou stimulant l'expression de marqueurs cutanés notamment choisis parmi la vimentine, la décorine, la fibroméduline, le biglycane et l'hyaluronsynthase.

3. Utilisation selon l'une quelconque des revendications précédentes, **caractérisées en ce que** la glucosamine est associée à un composé polyphénol.

4. Utilisation selon la revendication précédente, **caractérisée en ce que** le composé polyphénol est dérivé d'extraits végétaux choisis parmi les extraits de thé vert, de raisins tels que le *Vitis Vinifera,* de pin et notamment d'écorce de pin, de pomme, de myrtille, de houblon, de goyave, de cacao, de bois tels que le châtaignier, le chêne, le marronnier d'Inde et le noisetier.

5. Utilisation selon la revendication 4 ou 5, **caractérisée en ce que** le composé polyphénol se présente sous forme de proanthycyanidines, de flavonoïdes, de lignanes, de lignines, de stilbènes et de coumarines.

6. Utilisation selon la revendication précédente, **caractérisée en ce que** le composé polyphénol est un polyphénol catéchique choisi dans le groupe comprenant : catéchine, épicatéchine, gallactocatéchine, épigallactocatéchine sous forme monomère ou oligomère.

7. Utilisation selon la revendication précédente, **caractérisée en ce que** l'oligomère comprend de 2 à 14 unités catéchiques.

8. Utilisation selon l'une quelconque des revendications précédentes, **caractérisée en ce que** la glucosamine se présente sous forme d'un sel, sous forme acétylée et/ou sous forme polymérique.

9. Utilisation selon l'une quelconque des revendications précédentes, **caractérisée en ce que** la glucosamine se présente sous forme d'un sel choisi parmi le sulfate, le sulfate chloropotassique, le sulfate chlorosodique et le chlorhydrate de glucosamine.

10. Procédé cosmétique pour favoriser le rétablissement de l'homéostasie de l'épiderme et/ou pour limiter la dégradation des constituants matriciels du derme et/ou pour réduire le niveau d'expression de la MMP3 et/ou pour stimuler le cytosquelette des fibroblastes et/ou pour améliorer les propriétés contractiles des fibroblastes et/ou pour augmenter l'expression de marqueurs cutanés notamment choisis parmi la vimentine, la décorine, la fibroméduline, le biglycane et l'hyaluronsynthase et ainsi maintenir et/ou restaurer la fermeté cutanée induite par la ménopause, ledit procédé comprenant l'administration par voie orale d'une composition contenant de la glucosamine comprenant toutes ses formes salifiées, acétylées et/ou polymériques.

## Patentansprüche

1. Orale kosmetische Verwendung von Glucosamin, umfassend alle seine Salzformen, acetylierten Formen und/oder polymeren Formen zur Vorbeugung und/oder Behandlung von durch die Menopause verursachtem Verlust der Hautstraffheit.

2. Verwendung nach Anspruch 1, **dadurch gekennzeichnet, dass** Glucosamin als Aktivator der Kollagensynthese und/oder als Promotor der Wiederherstellung der epidermalen Homöostase und/oder als Mittel zur Hemmung der MMP3-Expression und/oder als Mittel zur Förderung der Fibroblastenkontraktilität und/oder zur Stimulierung der Expression von Hautmarkern, insbesondere ausgewählt aus Vimentin, Decorin, Fibromedulin, Biglycan und Hyaluronsynthase, wirkt.

3. Verwendung nach einem der vorhergehenden Ansprüche, **dadurch gekennzeichnet, dass** Glucosamin mit einer Polyphenolverbindung kombiniert wird.

4. Verwendung nach dem vorhergehenden Anspruch, **dadurch gekennzeichnet, dass** die Polyphenolverbindung aus Pflanzenextrakten gewonnen wird, die aus Extrakten von grünem Tee, Trauben wie *Vitis Vinifera,* Kiefern- und insbesondere Kiefernrinde, Apfel, Heidelbeere, Hopfen, Guave, Kakao, Holz wie Kastanie, Eiche, Rosskastanie und Haselnuss ausgewählt sind.

5. Verwendung nach Anspruch 4 oder 5, **dadurch gekennzeichnet, dass** die Polyphenolverbindung in Form von Proanthycyanidinen, Flavonoiden, Lignanen, Ligninen, Stilbenen und Cumarinen vorliegt.

6. Verwendung nach dem vorhergehenden Anspruch, **dadurch gekennzeichnet, dass** die Polyphenolverbindung ein katechisches Polyphenol ist, ausgewählt aus der Gruppe bestehend aus: Catechin, Epicatechin, Gallactocatechin, Epigallactocatechin, Epigallactocatechin in monomerer oder oligomerer Form.

7. Verwendung nach dem vorhergehenden Anspruch, **dadurch gekennzeichnet, dass** das Oligomer 2 bis 14 katechische Einheiten umfasst.

8. Verwendung nach einem der vorhergehenden Ansprüche, **dadurch gekennzeichnet, dass** das Glucosamin in Form eines Salzes, in acetylierter Form und/oder in polymerer Form vorliegt.

9. Verwendung nach einem der vorhergehenden Ansprüche, **dadurch gekennzeichnet, dass** Glucosamin in Form eines Salzes vorliegt, ausgewählt aus Sulfat, Chlorkaliumsulfat, Chlornatriumsulfat und Glucosaminhydrochlorid.

10. Kosmetisches Verfahren zur Förderung der Wiederherstellung der epidermalen Homöostase und/oder zur Begrenzung des Abbaus der Matrixbestandteile der Dermis und/oder zur Verringerung des Expressionsgrades von MMP3 und/oder zur Stimulierung des Zytoskeletts der Fibroblasten und/oder zur Verbesserung der kontraktilen Eigenschaften der Fibroblasten und/oder zur Erhöhung der Expression von Hautmarkern, insbesondere ausgewählt aus Vimentin, Decorin, Fibromedulin, Biglycan und Hyaluronsynthase, und dadurch Aufrechterhaltung und/oder Wiederherstellung der durch die Menopause induzierten Hautfestigkeit, wobei das Verfahren die orale Verabreichung einer Glucosamin enthaltenden Zusammensetzung umfasst, die alle ihre Salzformen, acetylierten Formen und/oder polymeren Formen umfasst.

## Claims

1. Cosmetic oral use of glucosamine comprising all its salified, acetylated and/or polymeric forms in the prevention and/or treatment of loss of skin firmness induced by menopause.

2. Use according to claim 1, **characterized in that** glucosamine acts as an activator of collagen synthesis and/or as a promoting agent for the restoration of epidermal homeostasis and/or by inhibiting the expression of MMP-3 and/or as an agent promoting the contractility of the fibroblasts and/or stimulating the expression of cutaneous markers chosen in particular from vimentin, decorin, fibromedulin, biglycan and hyaluron synthase.

3. Use according to one of the preceding claims, **characterized in that** the glucosamine is associated with a polyphenol compound.

4. Use according to the preceding claim, **characterized in that** the polyphenol compound is derived from vegetal extracts selected from the extracts of green tea, grapes such as *Vitis Vinifera,* pine and especially pine bark, apple, blueberry, hops, guava, cocoa, wood such as chestnut, oak, horse chestnut and hazel.

5. Use according to claim 4 or 5, **characterized in that** the polyphenol compound is in the form of proanthycyanidines, flavonoids lignans, lignins, stilbenes and coumarins.

6. Use according to the preceding claim, **characterized in that** the polyphenol compound is a catechol polyphenol chosen from the group comprising; catechin, epicatechin, gallocatechin, epigallactocatechin in monomer or oligomer form.

7. Use according to the previous claim, **characterized in that** the oligomer comprises from 2 to 14 catechitical units.

8. Use according to any one of the preceding claims, **characterized in that** the glucosamine is in the form of a salt, in acetylated form and/or in polymeric form.

9. Use according to any one of the preceding claims, **characterized in that** the glucosamine is in the form of a salt selected from sulfate, chloropotassic sulfate, chlorosodium sulfate and glucosamine hydrochloride.

10. Cosmetic process to favor the reestablishment homeostasis of the epidermis and/or to limit the degradation of the matrix constituents of the dermis and/or to reduce the level of expression of MMP3 and/or to stimulate the fibroblast cytoskeleton and/or to improve the property of the contractility of the fibroblasts and/or to increase the expression of cutaneous markers chosen, in particular, from vimentin, decorin, fibromedulin, biglycan and hyaluron synthase, and thus to maintain skin firmness and/or to restore its loss induced by the menopause, wherein the process comprises the oral administration of a composition containing glucosamine comprising all its salified, acetylated and/or polymeric forms.
